# EUROPEAN PATENT APPLICATION

(11) **EP 2 705 797 A2**
(43) Date of publication of application: **12.03.2014**
(21) Application number: 12779668.8
(22) Date of filing: 04.05.2012
(51) Int. Cl.: A61B 6/14

(54) **MARKER FOR IMAGE INFORMATION REGISTRATION**

(30) Priority: 04.05.2011 KR 20110042392
(71) Applicant: Yi, Tae-Kyoung, Seocho-gu Seoul 137-842 (KR); Jung, Je Kyo, Seoul 152-848 (KR)
(72) Inventor: Yi, Tae-Kyoung, Seocho-gu Seoul 137-842 (KR); Jung, Je Kyo, Seoul 152-848 (KR)
(74) Representative: Hoeger, Stellrecht & Partner Patentanwälte
(86) International application number: PCT/KR2012/003524
(87) International publication number: WO 2012/150843

(57) **Abstract**

Disclosed is a marker for image information matching. A marker for image information matching in accordance with an embodiment of the present invention is installed in an oral cavity of a patient to provide a reference point for obtaining image information on an oral tissue of the patient.

## Description

### [Technical Field]

The present invention relates to a marker and, more particularly, to an intraoral marker for the synchronization of three dimensional image data, which facilitates the production of a surgical guide used to synchronize coordinate systems between image information data on an oral tissue of a patient and image information data included in a model copied from the oral tissue of the patient or a processing machine on which the model copied from the oral tissue is mounted, thus accurately guiding an intraoral surgical procedure.

### [Background Art]

In general, imaging technologies in dental procedures are commonly used to guide the dental procedures as well as to establish diagnosis and procedure plans. The imaging technologies can be used in the design of the shape of prosthesis or in the production of a procedure guide device for an implant procedure and can be utilized in various fields such as gnathoplasty that requires a relative movement between maxilla and mandible.

In particular, in a dental implant procedure, in order to produce a surgical guide for guiding the implant procedure, which is used to guide the implantation position, direction, and depth of an implant for an intraoral alveolar bone, image data including anatomical information on the patient such as CT or MRI, oral cavity image information on the patient, are necessarily required, and image data of an impression model having the shape of an oral tissue of the patient may be separately required.

The surgical guide for guiding the implant procedure should have a negative pattern corresponding to the gingiva, teeth, and bone, and the shape of the surgical guide should be designed using an image containing anatomical information such as CT. However, the need for the surgical guide for guiding the implant procedure adapted to the patient's bone is limited by the demand for performing the surgical procedure without surgical incision of the gingiva if possible. For such reasons, the surgical guide for guiding the implant procedure should be designed and produced based on a suitable complementary shape corresponding to the gingiva or teeth. However, the image containing anatomical information such as CT or MRI may have a low resolution or in the case of a metal prosthesis, image impurities or distortions such as metal artifacts may occur, and thus a method for obtaining a more improved suitable complementary shape is required.

To obtain such a suitable complementary shape, a model copied from the oral cavity can be directly used or data obtained by three-dimensionally scanning the oral cavity can be used.

Among them, when the surgical guide for guiding the implant procedure is produced by bringing it into direct contact with an impression model copied using a dental impression material in the oral cavity of the patient, it is necessary to perform an additional drilling process at specified position, direction, and depth on a plaster model or a surgical guide for guiding the implant procedure formed on the plaster model to form a guide drill hole used to guide a drill for implant implantation in a body portion of the surgical guide for guiding the implant procedure, and a 5-axis machine has been used for such a drilling process.

The 5-axis machine itself has a coordinate system, and it is necessary to synchronize the coordinate system of the 5-axis machine with a reference point of the impression model.

In other words, the anatomical structure in which the implant is implanted in the patient's oral cavity is the alveolar bone, and the implantation position, direction, and depth are determined based on image information (CT) containing three-dimensional image information on the alveolar bone. Moreover, in order to produce a surgical guide for guiding the implant procedure with a high degree of accuracy, it is necessary to perform a process of matching the anatomical image information such as CT with the image information on the impression model.

Here, the surface data or shape of the impression model should be used to design or form the suitable complementary shape of the surgical guide for guiding the implant procedure, and a large amount of three-dimensional surface information on the copied model is required. That is, when three-dimensional numerical information on the position, direction, and depth of the implant determined based on the anatomical image information is implanted or coordinate-transformed into image information obtained from the copied model and then subjected to coordinate transformation again based on coordinate information on a site to be mounted on a processing machine, separately obtained from the copied model, the three-dimensional numerical information on the position, direction, and depth of the implant determined based on the anatomical image information can be converted into numerical information on the processing machine.

In the production of the above-described surgical guide for guiding the implant procedure, the process of matching different image information on the oral tissue and the copied model would play an important role in the production process. Moreover, in addition to the purpose for the production of the surgical guide for guiding the implant procedure, the matching of different image information on the oral tissue and the copied model can be used to more clearly distinguish the boundary of the patient's gingiva in the oral cavity in the intraoral anatomical image information. Accordingly, for the above reasons, the development of a method for implementing the matching of the anatomical image information and the different image information on the copied model at a high degree of accuracy or an apparatus and devices for the implementation of the method can be considered to be very important.

In order to match different image information, it is necessary to extract a common element (hereinafter referred to as a matching common element) required for the matching between two images. Image information on the tooth (including the prosthesis) or gingiva may be obtained from the image information on the copied model, and the shape of the alveolar bone or tooth (including the prosthesis) is mainly obtained from the intraoral anatomical image information. If there are a lot of image noises as the tooth is made of metal prosthesis, if the image accuracy of the image of the shape of the tooth is low, or in the case of an edentulous patient, it is difficult to use the matching common element. Moreover, if the gingiva is designated as the matching common element, the surface information on the gingiva cannot be readily obtained from the anatomical image information such as CT, and it is necessary to obtain image information in a state where the cheek is swollen in the oral cavity, a radiological contrast agent is retained in the oral cavity, or an additional intraoral device for incision of soft tissue is mounted, or to take an image by applying or adapting a radiological contrast agent, which is very troublesome.

In the information matching and coordinate synchronization required for the production of the surgical guide for guiding the implant procedure, a surgical guide in the form of thermoplastic resin for overlying gingiva and tooth has been used, but it is necessary to embed a plurality of radiopaque materials into a plurality of drilled holes on the surface of the surgical guide and apply the surgical guide in the oral cavity of the patient, thus taking CT images, which increases the number of processes and the time for each process before the CT scanning.

Moreover, in the case of a marker that is simply overlaid in the oral cavity of the patient or fixed by biting between the occlusal surface of the maxilla and mandible, the reproducibility of the mounting position in the oral cavity is significantly reduced or the fixed site is vulnerable, which makes it impossible to securely fix the marker, and thus the development of technical measures to cope with these problems is required.

### [Disclosure]

### [Technical Problem]

Embodiments of the present invention are to ensure more accurate three-dimensional information matching and coordinate synchronization required for simulation of implantation of an implant and for production of a surgical guide for guiding an implant procedure using a marker that provides a reference point with respect to image information on an oral tissue of a patient.

Embodiments of the present invention are to provide a marker that provides a reference point with respect to image information on an oral tissue of a patient and to allow a plaster model and a processing machine to share data included in the marker, thus easily matching coordinate systems of the plaster model and the processing machine.

Embodiments of the present invention are to obtain necessary image information by easily installing a marker including an outer cover on a tooth by means of the outer cover and to facilitate the matching with image information by means of an impression model.

Embodiments of the present invention are to ensure more stable installation of a maker and to obtain image information with reduced vibration even during occlusion of maxillary and mandibular teeth.

### [Technical Solution]

An embodiment of the present invention provides a marker for image information matching, the marker being installed in an oral cavity of a patient and providing a reference point for obtaining image information on an oral tissue of the patient.

The marker may comprise: a base provided at the bottom thereof; and a body provided at the top of the base and made of a radiopaque material.

The marker may comprise an outer cover surrounding the outside of the body and having a tooth shape.

The base may comprise a concave-convex portion provided on the outside thereof to increase adhesion with a tooth missing portion.

The body may project toward the top of the base or comprise a groove formed in an upper surface projecting toward the top of the base.

The body may project upward within a range from 1 mm to 6 mm.

The body may have a shape selected from the group consisting of a circular cone, a circular truncated cone, a sphere, or a circular cylinder.

The body may further comprise an inclined portion inclined inwardly or outwardly from above to below.

The marker may have a density different from that of the outer cover.

The base may be attached to a lingual side, a buccal, or an occlusal surface in the oral cavity.

The marker may be bent vertically with respect to the outer cover.

The marker may have a circular upper surface or a groove.

### [Advantageous Effects]

According to the embodiments of the present invention, it is possible to obtain more accurate processing data on a position where an implant is implanted by matching CT data of a patient with information included in three-dimensional data extracted from an impression model and by then matching the information included in the three-dimensional data of the impression model with a reference point of a processing machine.

According to the embodiments of the present invention, it is possible to guide accurate implantation of an implant by obtaining accurate information on an oral tissue of the patient.

According to the embodiments of the present invention, other than the case where the marker is located on the occlusal surface, it is possible to obtain stable CT data during occlusion of maxillary and mandibular teeth.

### [Description of Drawings]

FIG. 1 is a perspective view showing a marker in accordance with an embodiment of the present invention.
FIG. 2 is a longitudinal cross-sectional view of a marker in accordance with an embodiment of the present invention.
FIG. 3 is a perspective view showing a state where a marker for image information matching in accordance with an embodiment of the present invention is installed on a tooth missing portion of a patient.
FIGS. 4 to 6 are perspective views showing various examples of the marker in accordance with an embodiment of the present invention.
FIG. 7 is a perspective view showing a state where a marker for image information matching in accordance with another embodiment of the present invention is installed on a tooth missing portion of a patient.
FIGS. 8 and 9 are perspective views showing various examples of the marker in accordance with another embodiment of the present invention.
FIG. 10 is a perspective view showing a state where a marker in accordance with the present invention is installed on a tooth missing portion of a patient.
FIG. 11 is a view simply showing a relationship between CT data of a patient, CT data of an impression model, and a processing machine in a state where a marker in accordance with the present invention is installed.
FIG. 12 is a perspective view showing a stent with a bushing and an implant in accordance with an embodiment of the present invention.

### [Mode for Invention]

Hereinafter, preferred embodiments of the present invention will be described in detail with reference to the accompanying drawings.

Prior to description of a marker for image information matching in accordance with an embodiment of the present invention, it should be noted that the marker (an intraoral marker) of the invention is different from a surgical guide including a marker.

In order to include data information that the marker generally possesses in a tissue image in an oral cavity, the marker is formed or attached or a separate surgical guide having a negative pattern corresponding to the tooth or gingiva is fabricated.

It is apparent that such a separate surgical guide having a marker should be completed prior to a process of obtaining anatomical image information on an oral tissue of a patient, and for this reason the process of obtaining the anatomical image information from the patient is delayed.

According to the present invention, the marker is attached directly in the oral cavity to apply the marker in the oral cavity without preparing a conventionally used guide, and during the direct attachment of the marker to the oral tissue, the marker of the present invention is generally attached to the oral tissue of the patient. Here, the number of markers attached is three and may be increased by the number of missing teeth according to circumstances. However, it is important that the basic number of markers is three, and the reason for the minimum number is to provide three-dimensional information matching and coordinate synchronization, which is the final object of the marker of the present invention.

That is, the anatomical image information on the oral tissue of the patient is obtained in a state where three or more markers are attached or fixed in the oral cavity of the patient and then a simulated surgical procedure such as implant implantation is planned based on the anatomical image information containing information on the markers.

Upon completion of the simulated surgical procedure, three-dimensional numerical information (hereinafter referred to as surgical procedure information) including the simulated surgical procedure information is newly generated and recorded in a database, and then three-dimensional numerical information on a reference point for each marker is generated.

Three-dimensional reference information on each marker (hereinafter referred to as reference information) is combined with the surgical procedure information in the same relationship set, and the simulated surgical procedure and the reference point generation can be easily performed on the same simulation surgical procedure program without changing coordinate systems.

Previously, the anatomical image information is obtained in a state where the markers are attached or fixed in the oral cavity of the patient, and then an impression material is applied to the oral tissue of the patient to form a model (including a plaster model) copied from the oral tissue. Here, it should be noted that since the impression is obtained in a state where the markers are attached or fixed, the shape of the markers is successively integrated with the plaster shape of the tooth or gingiva in the copied model due to the negative pattern of the markers in the impression material.

Then, the three-dimensional image information on the copied model is obtained, and copy reference points are individually extracted from the copied marker shape formed on the copied model. Then, when coordinate transformation is performed by establishing a corresponding relationship between the copy reference points and the reference points in the anatomical image information, the anatomical image information and the three-dimensional information of the copied model are naturally matched. Here, it is apparent that the image information obtained from the copied model should have a relationship set with the surface data of the oral tissue, the complementary shape of a processing/transfer device which will be subjected to coordinate synchronization, and the copy reference points extracted from the copied marker shape formed on the copied model.

In the case of the surface data of the copied model, which is grouped into the same set with the copy reference points and subjected to coordinate synchronization, the matching of image information is achieved, and thus it is specially referred to as "image matching".

When the object which is grouped into the same set with the copy reference points and subjected to coordinate synchronization is a complementary shape, with which the copied model is combined with the processing/transfer device, or numerical information extracted therefrom, the coordinate system in the simulated surgical procedure program and the coordinate system of the processing/transfer device can be synchronized, and thus it is specially referred to as "coordinate synchronization".

Here, among the coordinate synchronization methods, even when the complementary shape, with which the copied model is combined with the processing/transfer device, is unknown, substantial coordinate synchronization can be realized. Moreover, after the copied model is fixed to a specific position of the processing/transfer device, the coped model may follow an intrinsic coordinate system of the processing/transfer device, and when a probe or scanner that recognizes the copied marker formed on the copied model, the coordinate synchronization can also be realized on the same principle. However, it should be noted that the markers used in the present invention should be in a relative corresponding relationship in both the oral cavity and the copied model.

The above-described relative corresponding relationship means that the relative position relationship between the markers attached or fixed in the oral cavity is the same as that on the copied model or is reproduced within the range of copy accuracy.

In view of the above definition, a pick-up impression commonly used in dental clinics or a method using the same may be used to form the markers in the oral cavity or on the copied model.

That is, in the case where a surgical attachment such as dental resin is formed on the tooth when the marker is attached in the oral cavity, if a copied model is formed with an impression after obtaining anatomical images in a state where the marker is attached or fixed in the oral cavity and then detaching the marker therefrom, an attachable resin is formed together with the oral cavity. Here, a negative pattern is formed on the attachable resin at the time when the markers are attached or fixed, and thus the markers can be located on the copied model with high reproducibility.

Like the above-described attachable resin, a base that allows the markers to be attached or fixed in the oral cavity, remains after the markers are removed, and has a copied shape on the copied model during the impression is referred to as an "addition-type attachable base".

Moreover, as a variation of the addition-type attachable base, a base that is removed at the same time when the markers are removed from the oral cavity and can be removed while maintaining the surface which is in contact with the oral cavity at the time when it is attached or fixed in the oral cavity is referred to as an "addition-type detachable base". Examples of dental practice include resin for temporary dental prosthesis, impression silicon, impression putty, restorative resin, etc.

As such, whether the impression is made in a state where the markers are attached or fixed in the oral cavity or whether the impression is made using the "addition-type attachable base" or the addition-type detachable base, the markers used in the present invention are advantageously applicable to the oral tissue or copied model in a very simple way or without the necessity of preparing additional guides during the step of applying the markers to the oral cavity or during the steps prior to obtaining the anatomical images.

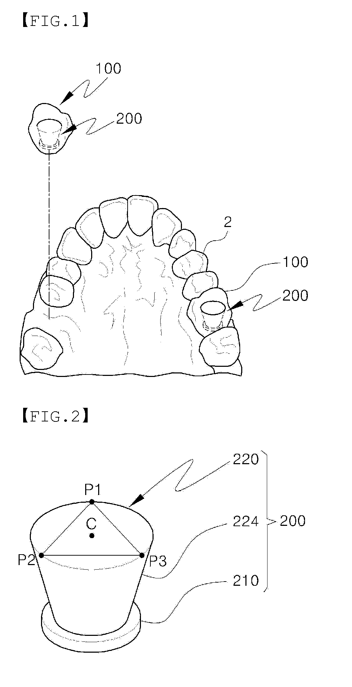

To this end, the configuration of a marker for image information matching in accordance with an embodiment of the present invention will be described with reference to the drawings. FIG. 1 is a perspective view showing a marker in accordance with an embodiment of the present invention, and FIG. 2 is a longitudinal cross-sectional view of a marker in accordance with an embodiment of the present invention.

Referring to FIGS. 1 and 2, a marker 200 comprises a body 220 of a circular shape, an inclined portion 224 inclined toward the bottom of the body 220, and a base 210 provided with a concave-convex portion 212 on a lower surface of the marker 200 to increase adhesion with a tooth missing portion.

The base 210 is in surface contact with the outer circumference of a tooth and may be a rectangular shape or a circular plate shape as shown in the figures.

The marker 200 has a center point C in the middle of the upper surface, and the center point C becomes a reference point during implantation of coordinate data of a coordinate synchronization object. Here, the coordinate synchronization object corresponds to the marker whose coordinate data defining numerical information or image information is to be extracted. The center point C corresponds to a starting point for the recognition of the coordinate data and also includes a concept of a starting point of a three-dimensional coordinate axis as well as a basic concept of a one-dimensional point. That is, the center point C may be the center of a triangle generated by connecting three points defined in the circumferential direction of the marker 200.

Coordinate data of the marker 200 corresponding to the coordinate synchronization object defines the numerical information or image information of the coordinate synchronization object. The data that defines the image information corresponds to data that defines the shape of the coordinate synchronization object, and the data that defines the numerical information refers to other coordinate information expressed in the coordinate synchronization object. For example, processing vectors (i.e., vector information that defines the processing direction, depth, and shape) expressed in the image information data by a computer simulation correspond to the numerical information data.

The concave-convex portion 212 provided in the marker 200 may have various shapes and, for example, may have a concave shape or a convex shape, but not limited thereto. When an outer cover 100 included in the marker 200 is attached to a tooth missing portion, the concave-convex portion 212 serves to increase the adhesion as an adhesive such as wax is applied to the base 210 and serves to prevent the outer cover 100 from being shaken or twisted in the oral cavity of the patient. The adhesive may include any adhesive that is harmless to human body and is not limited to particular materials.

The body 220 of the marker 200 may have a flat upper surface, and the upper surface of the marker 200 is extracted from a triangle defined as three points in the circumferential direction. That is, when a triangle is defined as three points P1, P2, and P3 in the circumferential direction of the body 220, a center point C of the three points P1, P2, and P3 can be easily extracted, which makes it possible to establish a three-dimensional coordinate system based on the center point C. Here, the reason that the minimum number of coordinates is three is that a circle cannot be formed by two coordinates, and in the case of more than three coordinates, they may have an irregular pattern that does not form a circle.

The marker in accordance with an embodiment of the present invention may be integrally formed with a marker having a tooth shape, which will be described with reference to the drawings. For reference, a patient's teeth shown in FIG. 1 are in a partially edentulous jaw.

Referring to FIG. 1, an outer cover 100 with a marker is installed in the oral cavity of the patient, and the outer cover 100 has a tooth shape. In this embodiment, the outer cover 100 has a molar shape, but it may have an incisor, canine, or premolar shape. The outer cover 100 may be installed on a tooth missing portion in the oral cavity.

The outer cover 100 may have different sizes in terms of patients' ages. For example, the outer cover used in an adult and the outer cover used in a child may be different sizes. However, the present embodiment will be described with reference to the size and structure shown in FIG. 2 for convenience of description.

The marker 200 may be integrally formed with the outer cover 100 or may be selectively detachable, and in the case of a detachable marker 200, the marker 200 can be provided with various shapes.

The reason that the marker 200 is integrally formed with the outer cover 100 is to obtain more accurate information on the reference points in the oral tissue of the patient, thus ensuring accurate CT data of the patient through CT scanning in a state where the outer cover 100 is installed. Moreover, in the case where the outer cover 100 has a tooth shape and is installed on a tooth missing portion, it is possible to prevent interference with adjacent normal teeth.

Various examples of the marker in accordance with an embodiment of the present invention will be described with reference to the drawings, in which these various markers have shapes that facilitate the extraction of the center point C.

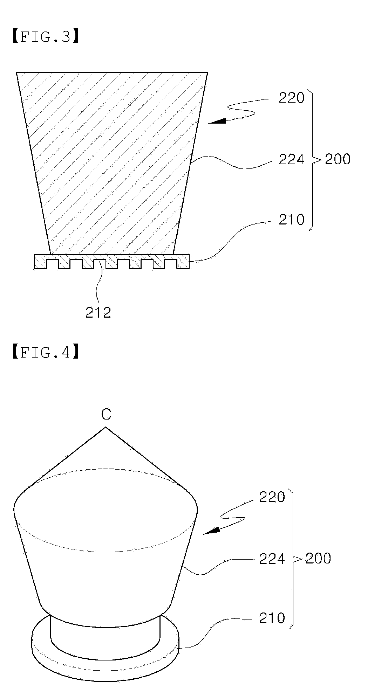

Referring to FIGS. 4 to 6, the marker 200 may have a circular cone shape, in which the upper surface of the body 220 projects upward. In the case where the upper surface of the body 220 has such a shape, the peak of the body 220 is the center point C. As such, in the case where the marker 200 has a circular cone shape, it is possible to advantageously obtain accurate data of the center point C. The body 220 may project upward within a range from 1 mm to 6 mm.

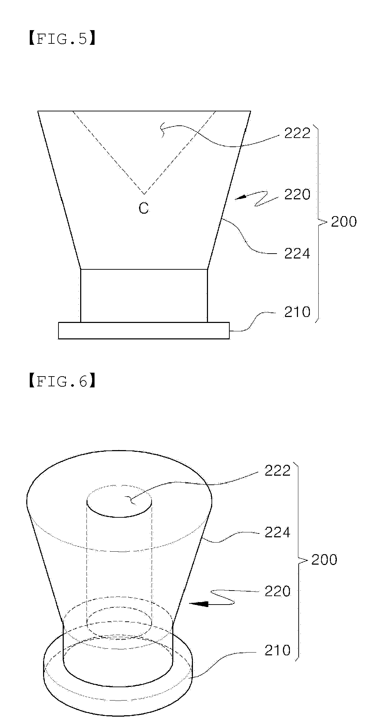

Referring to FIG. 5, the marker 200 has a V-shape groove 222 formed in the middle of the inside of the body 220, and the bottom center of the groove 222 is the center point C. Unlike the marker illustrated in FIG. 1, the center of the triangle defined as three points in the circumferential direction is not defined as the center point, but the center point C is provided on the groove 222 formed in the inside of the marker 200.

Referring to FIG. 6, the marker 200 may be formed in any desired shape such as a circular truncated cone, a sphere, or a circular cylinder as well as the above-described circular cone shape or grooved shape. As such, the markers 200 formed in various shapes can facilitate the extraction of the center point C from the outline of the upper and lower surfaces of the body 220, thus increasing the accuracy of the matching.

Referring to FIGS. 2 to 6, the marker 200 in accordance with an embodiment of the present invention has the inclined portion 224 whose side is inclined inwardly from above to below. The reason that the inclined portion 224 is provided is that, in the case where both the upper and lower surfaces of the marker 200 have the same diameter, it is necessary to determine one of the centers of the upper and lower surfaces to be set as the reference point prior to the data extraction and, during matching of the reference points, the centers of different marker surfaces may be set as the reference points, thus causing an error in the matching or an inaccurate matching.

Accordingly, it is possible to ensure accurate matching by the different shapes of the upper and lower surfaces due to the inclined portion 224, thus performing image matching.

The marker 200 in accordance with an embodiment of the present invention may have a density different from that of the outer cover 100. The marker 200 is made of a radiopaque material to show its shape during CT scanning and may have a density from 1 to 5.0 g/cm³, for example, to be distinguished from the oral tissue.

In the case where the density of the marker 200 is 1 g/cm³ or lower, it is similar to an image value of soft tissue, and thus the marker 200 may be lost during segmentation, whereas in the case where the density is 4.5 g/cm³ or higher, metal artifacts may occur to deteriorate the quality of the image unless the current and voltage values for the radiation are increased, and thus it is preferable that the maker 200 has the above density range, but not limited thereto.

The configuration of a marker for image information matching in accordance with another embodiment of the present invention will be described with reference to the drawings.

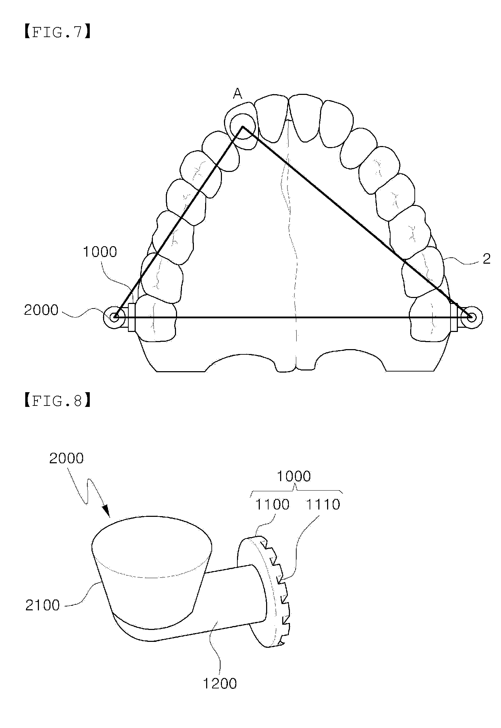

Referring to FIG. 7, a marker 2000 is integrally formed with an outer cover 1000 provided on a lingual or buccal side of a tooth and is provided for image matching of the outer cover 1000.

Like the above-described marker 200, the markers 2000 are provided on teeth arranged in the oral cavity of a patient or, in particularly, disposed on a lingual or buccal side of a molar and on an occlusal surface.

When the markers 2000 are provided at positions A, the area formed by an imaginary triangle connecting the respective center points covers a dental arch as widely as possible, thus enabling more accurate image matching. The reason for this is that, if the area formed by the imaginary triangle is smaller and thus a tooth to be processed is located outside the imaginary triangle, an interpolation method for obtaining data of the tooth to be processed is applied to the area of the imaginary triangle, which increases the possibility of errors at the position, depth, and height of the tooth, resulting in inaccurate information.

To this end, it is preferable that a total of three markers 2000 in accordance with another embodiment of the present invention are provided in the oral cavity of the patient.

For example, first and second markers are provided on left and right molars and the third marker is provided on an incisor such that the triangle connecting the respective center points C has an area that can cover the teeth of the patient as much as possible, which makes it possible to obtain more accurate image information on the oral tissue of the patient.

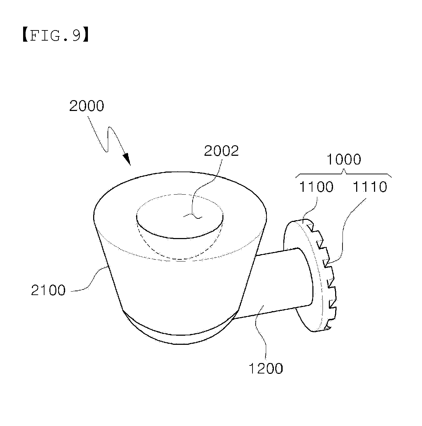

Referring to FIGS. 8 and 9, the marker 2000 in accordance with another embodiment of the present invention is disposed vertically with respect to the outer cover 1000 attached to the outside of a tooth 2. The outer cover 1000 is provided with a support base 1100 including a concave-convex portion 1110 formed on the outside thereof to increase adhesion with the tooth 2. The support base 1100 may be a circular plate shape or a round shape having a curvature similar to an outer circumferential curvature of the tooth.

As previously described, the concave-convex portion 1110 may have a concave shape or a convex shape, but not limited thereto. When the outer cover 1000 included in the marker 2000 is attached to the tooth, the concave-convex portion 1110 can increase the adhesion as an adhesive such as wax is applied to the support base 1100 and prevent the outer cover 1000 from being shaken or twisted in the oral cavity of the patient.

A connection piece 1200 is provided between the support base 1100 and the marker 200, and the connection piece 1200 has a predetermined length and extends outwardly. The extension length of the connection piece 1200 is not particularly limited, but is preferably 1 cm to several cm such that the marker 2000 may be disposed adjacent to the tooth 2.

The marker 2000 may have a circular upper surface or a groove 2002, or although not shown in the figures, may have a circular cone shape. In the case where the marker 2000 is configured in this manner, the center of the triangle defined as three points in the circumferential direction is not defined as the center point, but the center point C is provided on the groove 2002 formed in the inside of the marker 2000.

The marker 2000 may be formed in any desired shape such as a circular cone, a circular truncated cone, a sphere, or a circular cylinder as well as the above-described shapes. As such, the markers 2000 formed in various shapes can facilitate the extraction of the center point C from the outline of the maker 2000, thus increasing the accuracy of the matching.

The marker 2000 in accordance with another embodiment of the present invention has an inclined portion 2100 whose side is inclined inwardly from above to below. The reason that the inclined portion 2100 is provided is that, in the case where both the upper and lower surfaces of the marker 2000 have the same diameter, it is necessary to determine one of the centers of the upper and lower surfaces to be set as the reference point prior to the data extraction and, during matching of the reference points, the centers of different marker surfaces may be set as the reference points, thus causing an error in the matching or an inaccurate matching.

Accordingly, it is possible to ensure accurate matching by the different shapes of the upper and lower surfaces due to the inclined portion 2100 of the marker 2000, thus performing image matching.

Next, the use state of the above-described marker for image information matching in accordance with the present invention will be described with reference to the drawings. For convenience of description, it will be described that the marker is inserted in a mandibular tooth.

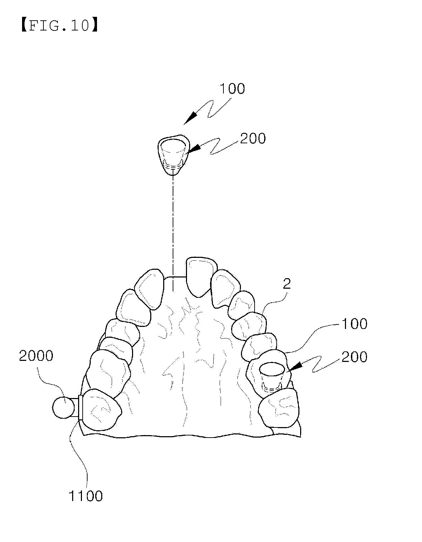

Referring to FIG. 10, an operator installs the marker 200, 2000 on a tooth or a tooth missing portion. The marker is installed on left and right molars and an incisor, respectively and, in the case where the marker is installed on the tooth missing portion, the outer cover 100, 1000 having a shape corresponding to that of the tooth missing portion is provided on the tooth missing portion.

As the outer cover 100 having a shape similar to that of the tooth is installed on the tooth missing portion, it is possible to prevent interference with adjacent teeth.

Moreover, the marker 200 is inserted in a state where a separate adhesive is applied to the base 210, and due to the concave or convex shape formed in the concave-convex portion 212, the adhesion on the surface can be more improved. Accordingly, the outer cover 100 does not shake from side to side or move from the initial position and is thus maintained in a state where the positional stability is maximized.

The marker 2000 is attached to the tooth with the support base 1100 interposed therebetween, whose outer surface is applied with an adhesive, and due to the concave-convex portion provided on the support base 1100, the adhesion between the marker 2000 and the tooth can be stably maintained.

In such a state where the markers 200, 2000 are all installed in the oral cavity of the patient, the operator performs CT scanning to obtain data of the markers 200, 2000. The CT scanning is performed during occlusion of maxillary and mandibular teeth in a state where the markers are inserted in the tooth missing portions. As a result, the CT scanning is made in a state where the markers 200, 2000 do not move or the vibration thereof is minimized, thus ensuring more accurate data on the position, direction, and depth of the tooth missing portions.

Moreover, unlike the markers installed on the teeth, the markers 200, 2000 are attached to the lingual or buccal side to ensure the maximum area covering the teeth, thus obtaining more accurate data.

As mentioned above, the acquisition of the data on the markers 200, 2000 is performed as follows. The processing vectors on the positions where the markers 200, 2000 are installed are obtained through CT scanning. The information on the markers 200, 2000 may be obtained by recognizing coordinates of the markers 200, 2000 in such a manner that a detection tip for coordinate detection is brought into contact with the upper surface of each marker 200, 2000, and any one of the two methods may be selectively used. In the present embodiment, for convenience of description, it will be described that the data on the markers 200, 2000 is obtained through the CT scanning.

Separately from the data obtained in a state where the markers 200, 2000 are inserted into the oral cavity of the patient in the above manner, an impression model having the same shape as maxillary and mandibular teeth is prepared by forming a negative pattern of the maxillary and mandibular teeth using a rubber impression material and then pouring plaster into the impression model.

The impression model has empty spaces corresponding to the tooth missing portions of the patient, and CT data of the impression model is obtained by performing CT scanning on the impression model in a state where the markers 200 are inserted into the empty spaces.

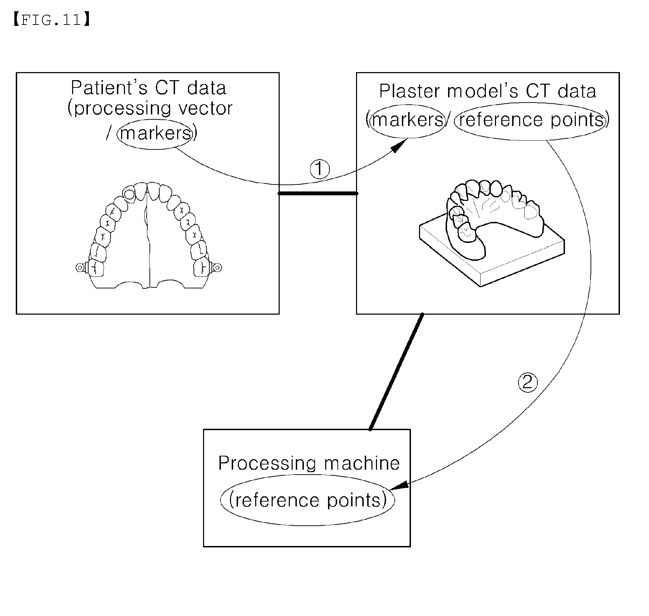

Referring to FIG. 11, the information on the markers and the processing vector information are included in the CT data of the patient, the information on the markers and information on reference points are included in the CT data of the impression model, and the information on the reference points is included in a processing machine. Here, the information on the reference points of the plaster model refers to the coordinates of a coordinate synchronization plate having a rectangular plate shape at the bottom of the impression model, and the reference points project toward the upper surface of the coordinate synchronization plate and are arranged in a triangle.

The reference points guide the impression model to be accurately positioned on the upper surface of the coordinate synchronization plate, and the CT data of the plaster model obtained in a state where the impression model is placed on the coordinate synchronization plate may be recognized as the coordinate system of the processing machine by means of the reference points.

When the marker information of the CT data is matched with the marker information of the CT data included in the impression model as shown in ① and the reference point information of the CT data included in the impression model is matched with the reference point information of the processing machine as shown in ②, the information on the markers installed in the patient is matched with the reference points of the processing machine and transferred to the coordinate system of the processing machine, thus obtaining accurate processing data.

The processing data may be simulated before performing implantation of an implant in the patient's oral cavity by a separate program included in the processing machine, thus minimizing the occurrence of errors during the implantation of the implant.

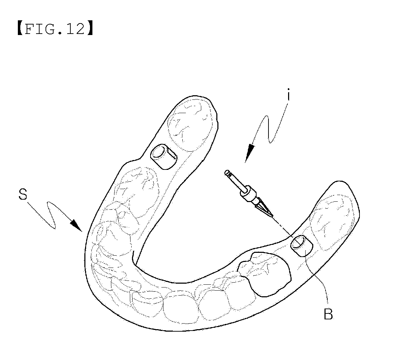

Referring to FIG. 12, an operator forms a through hole using a drill for drilling alveolar bone in the plaster model (not shown) where the implant is to be implanted and installs a bushing B in the through hole. The bushing B is disposed to project a predetermined length from the plaster model. Then, the operator applies resin to the plaster model to form a dental stent S and forms a through hole in the above-described bushing B.

Then, the operator inserts an implant (not shown) into the bushing B in a state where the dental stent S is located in the oral cavity of the patient and performs the implantation. As a result, the implant may be stably implanted at a predetermined depth and angle in a state where the implant is accurately located at the implantation position of the patient.

While the invention has been shown and described with reference to certain preferred embodiments thereof, it will be understood by those skilled in the art that various changes in form and details may be made therein without departing from the spirit and scope of the invention as defined by the appended claims. Therefore, the scope of the invention is defined not by the detailed description of the invention but by the appended claims, and all differences within the scope will be construed as being included in the present invention.

## Claims

1. A marker for image information matching, the marker being installed in an oral cavity of a patient and providing a reference point for obtaining image information on an oral tissue of the patient.

2. The marker of claim 1, wherein the marker comprises:
a base provided at the bottom thereof; and
a body provided at the top of the base and made of a radiopaque material.

3. The marker of claim 2, wherein the marker comprises an outer cover surrounding the outside of the body and having a tooth shape.

4. The marker of claim 2, wherein the base comprises a concave-convex portion provided on the outside thereof to increase adhesion with a tooth missing portion.

5. The marker of claim 2, wherein the body projects toward the top of the base or comprises a groove formed in an upper surface projecting toward the top of the base.

6. The marker of claim 5, wherein the body projects upward within a range from 1 mm to 6 mm.

7. The marker of claim 2, wherein the body has a shape selected from the group consisting of a circular cone, a circular truncated cone, a sphere, or a circular cylinder.

8. The marker of claim 3, wherein the body further comprises an inclined portion inclined inwardly or outwardly from above to below.

9. The marker of claim 3, wherein the marker has a density different from that of the outer cover.

10. The marker of claim 2, wherein the base is attached to a lingual side, a buccal, or an occlusal surface in the oral cavity.

11. The marker of claim 3, wherein the marker is bent vertically with respect to the outer cover.

12. The marker of claim 1, wherein the marker has a circular upper surface or a groove.
